(19) Europäisches Patentamt
European Patent Office
Office européen des brevets

(11) **EP 2 001 425 B1**

(12) **EUROPEAN PATENT SPECIFICATION**

(45) Date of publication and mention
of the grant of the patent:
**14.09.2011 Bulletin 2011/37**

(51) Int Cl.:
***A61F 13/15*** *(2006.01)*

(21) Application number: **07754456.7**

(22) Date of filing: **30.03.2007**

(86) International application number:
**PCT/US2007/007939**

(87) International publication number:
**WO 2007/123702 (01.11.2007 Gazette 2007/44)**

(54) **ABSORBENT ARTICLE COMPRISING A FIBROUS STRUCTURE COMPRISING SYNTHETIC FIBERS AND A HYDROPHILIZING AGENT**

SAUGFÄHIGER ARTIKEL MIT EINER FIBRÖSEN STRUKTUR MIT SYNTHETISCHEN FASERN UND EINEM HYDROPHILIERUNGSMITTEL

ARTICLE ABSORBANT COMPRENANT UNE STRUCTURE FIBREUSE CONSTITUÉE DE FIBRES SYNTHÉTIQUES ET AGENT HYDROPHILISANT

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **31.03.2006 US 788417 P**
**03.04.2006 US 788620 P**

(43) Date of publication of application:
**17.12.2008 Bulletin 2008/51**

(73) Proprietor: **The Procter & Gamble Company**
**Cincinnati, OH 45202 (US)**

(72) Inventors:
• **POLAT, Osman**
**Montgomery, Ohio 45249 (US)**
• **PHAN, Dean, Van**
**West Chester, Ohio 45069 (US)**
• **TROKHAN, Paul, Dennis**
**Hamilton, Ohio 45013 (US)**
• **CATALAN, Kemal, Vatansever**
**Cincinnati, Ohio 45255 (US)**
• **ULLMAN, Alan, Howard**
**Blue Ash, Ohio 45242 (US)**

(74) Representative: **Heide, Ute et al**
**Procter & Gamble European Service GmbH**
**Sulzbacher Strasse 40-50**
**65824 Schwalbach am Taunus (DE)**

(56) References cited:
**EP-A1- 0 438 114      WO-A-94/05243**
**GB-A- 2 269 603      US-A- 3 416 952**
**US-A- 5 356 405**

• **DATABASE WPI Section Ch, Week 198403 Derwent Publications Ltd., London, GB; Class A28, AN 1984-015055 XP002453249 & JP 58 208499 A (TEIJIN LTD) 5 December 1983 (1983-12-05)**

**Description**

FIELD OF THE INVENTION

The present invention relates to absorbent articles comprising fibrous structures comprising synthetic fibers. The synthetic fibers may further be associated with a hydrophilizing agent.

BACKGROUND OF THE INVENTION

Disposable absorbent articles, such as diapers, adult incontinence products, and feminine hygiene products, are well known in the art. Such disposable articles collect and retain urine, menses and fecal material deposited thereon by the wearer.

Fibrous structures, such as paper webs, are well known in the art and are in common use today for absorbent articles, for example, as topsheet material or as a core cover to enclose the absorbent core. Various natural fibers, including cellulose fibers, as well as a variety of synthetic fibers, have been employed in papermaking. Typical tissue paper may be comprised primarily of natural fibers. The overwhelming majority of the natural fibers used in tissue may be derived from trees. Many species may be used, including long fiber containing softwoods (conifer or gymnosperms) and short fiber containing hardwoods (deciduous or angiosperms).

Despite a broad range of natural fiber types, natural fibers derived from trees may be limiting when used exclusively in disposable tissue and towel products. Wood fibers may be high in dry modulus and relatively large in diameter, which may cause their flexural rigidity to be higher than desired for some uses. Such high-rigidity fibers may produce stiff non-soft tissue. Further, wood fibers can have the undesirable characteristic of having a relatively high stiffness when dry, which may negatively affect the softness of the product and may have low stiffness when wet due to hydration, which may cause poor absorbency of the resulting product. Wood-based fibers may also be limiting because the geometry or morphology of the fibers cannot be "engineered" to any great extent.

EP 0438114 A1 relates to hydrophilization of polyester articles. The polyester articles are rendered hydrophilic by providing the polyester article with a generally hydrophilic surface by contacting the polyester article with a copolymeric material while it is at a temperature above its melting point. The copolymeric material comprises a hydrophobic moiety and a hydrophilic moiety.

GB 2269603 A relates to fluff pulp, suitable for use in the absorbent core of an absorbent product such as a diaper or the like, comprises a blend of cellulosic fibres and synthetic heat-shrunk fibres, e.g. of polyester. The synthetic organic fibres may have a surface pretreatment to render them hydrophilic before they are incorporated into the blend with the cellulose fibres. One disclosed pretreatment is such as to improve the dispersibility of the fibres in water.

WO 94/05243 relates to absorbent articles, especially sanitary napkins, containing a flow regulator positioned between the topsheet and the absorbent core. In-use, fluid deposited on the topsheet is internally moved in the longitudinal direction by the flow regulator before being released to the absorbent core. By moving substantial amounts of fluid in the longitudinal direction the flow regulator provides a more effective use of the absorbent core.

US 5,356,405 relates to absorbent articles, especially sanitary napkins, containing fibers with intra-fiber capillary channels. In-use, the capillary channel fibers direct menses to a storage layer, thereby minimizing product failure and staining of undergarments. The capillary channel fibers can protrude into, or through, a topsheet to provide very transport of vaginal discharges.

The use of synthetic fibers that have the ability to thermally fuse to one another and/or to natural fibers is an excellent way to overcome the previously mentioned limitations of natural fibers. Wood-based natural fibers are not thermoplastic and hence cannot thermally bond to other fibers. Synthetic thermoplastic polymers can be formed into fibers with a range of diameters, including very small fibers. Further, synthetic fibers can be formed to be lower modulus than natural fibers. Thus, a synthetic fiber can be made with very low flexural rigidity, which may increase product softness. In addition, functional cross-sections of the synthetic fibers can be micro-engineered during the spinning process. Synthetic fibers can also be designed to maintain modulus when wetted, and hence webs made with such fibers may resist collapse during absorbency tasks. Further, the use of synthetic fibers can aid in the formation of a web and/or its uniformity. Accordingly, the use of thermally bonded synthetic fibers in tissue and towel products can result in a strong network of highly flexible fibers (good for softness) joined with water-resistant high-stretch bonds (good for softness and wet strength).

The use of synthetic fibers, however, may have some limitations. The synthetic fibers may have a general characteristic of being hydrophobic. As such, the suspension of the hydrophobic synthetic fibers in a fluid carrier during the papermaking process may result in a slurry in which the hydrophobic synthetic fibers have clumped together. A fibrous structure created from such a slurry may demonstrate areas of high stiffness when dry and low stiffness when wet. Thus, the benefits of utilizing synthetic fibers to maintain the modulus of the fibrous structure when wet may not be realized. Additionally, the hydrophobic character of the synthetic fibers may overcome the generally hydrophilic character of the natural fibers. This, in turn, may have a negative impact on the fibrous structure and may result in a decrease in absorbency and/or rate of absorption of the overall structure.

Fibrous structures are usually hydrophobic, as noted above. However, for many applications in hygiene products, such as absorbent articles, it is necessary to have hydrophilic fibrous structures. Therefore, the fibrous structure has to be treated accordingly.

One typical component of disposable absorbent articles is a core cover. A core cover may typically be a nonwoven material designed to contain the absorbent core and provide structural integrity when the absorbent core is wet or dry. The core cover may be a tissue wrap, or a nonwoven material which has been rendered hydrophilic.

A common method for rendering nonwoven fibrous structures hydrophilic is coating the surface of the nonwoven fibrous structure with hydrophilic surfactants. As this coating does not lead to a tight chemical bond between the nonwoven fibrous structure and the surfactant, the surfactant can be washed off during use when the absorbent article is wetted. The decrease in liquid strike-through time is a desirable effect when the fibrous structure is coated with surfactant. Liquid strike-through refers to liquid passing through the fibrous structure with liquid strike-through time referring to the time it takes for a certain amount of liquid to pass through the fibrous structure. However, as the surfactant is washed off when the coated fibrous structures are exposed to the liquid, the strike-through time in the next gush is increased. This results in performance reduction during use on diapers or other articles comprising such fibrous structures. Furthermore, at the same time as liquid strike-through time decreases due to use of surfactants, surface tension of the liquid, which was in contact with the fibrous structure, is reduced. This reduction is undesirable, because it can cause increased urine leakage in a diaper.

Another common method to render a fibrous structure hydrophilic is by applying corona and/or plasma treatment. Plasma is an ionized form of gas that can be obtained by ionizing a gas or liquid medium. Plasmas are widely used for the treatment of organic and inorganic materials to promote adhesion between various materials. Polymers that have chemically inert surfaces with low surface energies do not allow good coatings with bondings and adhesives. Thus, these surfaces are treated to make them receptive to bonding with other substrates, coatings, adhesives and printing inks. However, corona and plasma treatments lead to low coating durability upon storage of the treated material, i.e., hydrophilicity decreases over time.

A wide variety of hydrophilizing agents for use in domestic and industrial fabric treatment processes such as laundering, fabric drying in hot air clothes dryers, and the like, are known in the art and are conventionally referred to in those fields as "Soil Release Polymers" (SRP's) or "Soil Release Agents" (SRA's). Various oligomeric or polymeric hydrophilizing agents have been commercialized and are known for their use as soil release compounds in detergent compositions and fabric softener/antistatic articles and compositions. Hydrophilizing agents utilized in laundry applications generally are employed to pre- or post-treat woven fabrics. Woven fabrics pre-treated with hydrophilizing agents may exhibit stain guard characteristics while woven fabrics post-treated with hydrophilizing agents may exhibit stain release characteristics. The woven fabrics may be washed and re-washed and may retain their stain guard and stain release characteristics. Such hydrophilizing agents which comprise an oligomeric or polymeric ester "backbone" are sometimes referred to as "Soil Release Esters" (SRE's).

Hydrophilizing agents may also associate with synthetic fibers in a nonwoven fibrous structure. It has now been found that the use of a hydrophilizing agent to associate with the synthetic fibers of a nonwoven fibrous structure may have the ability to overcome one or more of the above mentioned disadvantages associated with the use of synthetic fibers. It has now been found that the association of the hydrophilizing agents with synthetic fibers may enable the synthetic fibers to display hydrophilic characteristics thus overcoming the general hydrophobic nature of the synthetic fibers. This may allow for the dispersion of the synthetic fibers throughout the nonwoven fibrous structure instead of clumping together and may help provide a more homogeneous distribution of the fibers in webs which also comprise natural fibers. A uniform distribution of synthetic fibers which have associated with hydrophilizing agents in combination with natural fibers may also result in a fibrous structure that is hydrophilic in nature. A fibrous structure that is hydrophilic in nature may exhibit an increase in the absorbency and/or rate of absorption of fluids. Therefore, the utilization of hydrophilizing agents may result in a positive impact on the absorbency and/or rate of absorption of the nonwoven fibrous structure.

Absorbent articles, such as diapers, adult incontinence products, and feminine hygiene products, are well known articles of staple manufacturing. Multiple attempts have been made to provide them with an overall good fit and with a high absorbent capacity. Modem absorbent articles may make use of absorbent polymer materials or so-called superabsorbent materials. The absorbent polymer materials are generally surrounded by nonwoven materials. Many nonwoven materials that incorporate synthetic fibers are hydrophobic. The performance of absorbent polymer materials surrounded by a hydrophobic fibrous structure may be negatively affected as fluid may not be absorbed as readily by the polymer material if repulsed by the surrounding hydrophobic fibrous structure.

It would be desirable to provide improved fibrous structures comprising synthetic fibers in association with hydrophilizing agents. It would be desirable to provide a fibrous structure in which the synthetic fibers exhibit hydrophilic characteristics. It would be desirable to provide a fibrous structure in which the synthetic fibers are dispersed throughout the fibrous structure. It would be desirable to provide a fibrous structure in which absorbency is not negatively impacted. It would be desirable to utilize such a fibrous structure in an absorbent article to enhance performance of the absorbent article. It would be desirable to provide an absorbent article in which the rate of absorption is acceptable to consumers of the absorbent article.

## SUMMARY OF THE INVENTION

The present invention relates to an absorbent article comprising a nonwoven fibrous structure comprising plurality of synthetic fibers and a hydrophilizing agent. The synthetic fibers and the hydrophilizing agent may comprise a durable association.

In one example of the present invention, an absorbent article comprising a nonwoven fibrous structure comprising 1) a plurality of synthetic fibers, wherein one or more (or each) of said synthetic fibers comprises a polymer, and 2) a hydrophilizing agent, wherein said polymer and said hydrophilizing agent comprise complementary segments that are associated with one another, is provided.

The synthetic fibers of the fibrous structure may comprise a polymer. The polymer and the hydrophilizing agent may comprise complementary segments that may associated with one another. At least one of the complementary segments may comprise a polyester segment. The polymer of the synthetic fiber may comprise material selected from the group consisting of polyesters, polyamides, polyhydroxyalkanoates, polysaccharides, and combinations thereof. The fibrous structure may further comprise a plurality of natural fibers.

The hydrophilizing agent may be a copolymer. The hydrophilizing agent may be selected from the group consisting of polyester, poly(ethoxylate), polyethylene oxide, polyoxyethylene, polyethylene glycol, polypropylene glycol, terephthalate, polypropylene oxide, polyethylene terephthalate, polyoxyethylene terephthalate, ethoxylate siloxane and combinations thereof. The hydrophilizing agent may have from about 1 to about 15 ethoxylated groups.

The nonwoven fibrous structure may further comprise binder material. The binder material may be selected from the group consisting of permanent wet strength resins, temporary wet strength resins, dry strength resins, latex binders and combinations thereof.

## BRIEF DESCRIPTION OF THE DRAWINGS

Figure 1 is a partial cut-away top plan view of a disposable absorbent article including an absorbent core and a core cover.
Figure 2 is a partial sectional view along 2-2 of one alternative embodiment of the absorbent core of the disposable absorbent article of Figure 2.

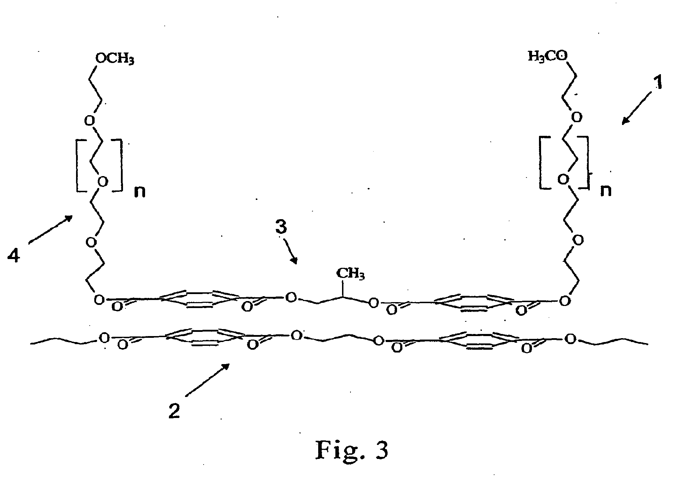

Figure 3 depicts an artist's conception of the association of a dimeric hydrophilizing agent and a synthetic fiber.

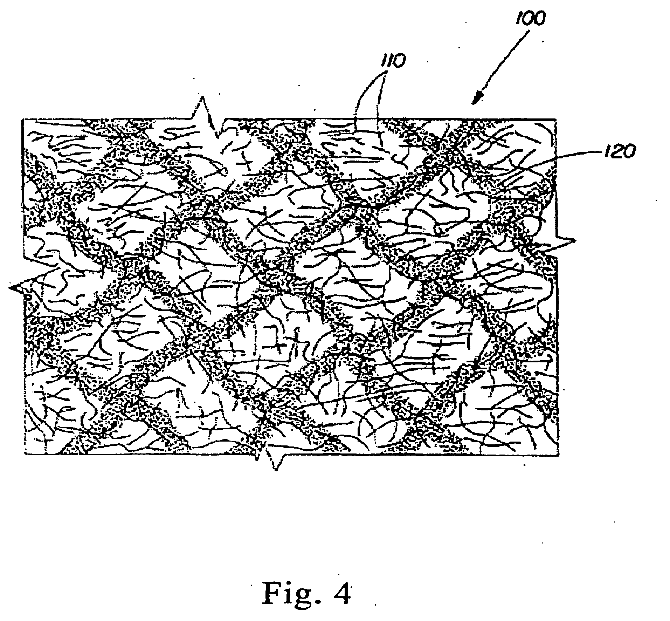

Figure 4 depicts a schematic plan view of an embodiment of a fibrous structure of the present invention in which the synthetic fibers are distributed in a non-random pattern.

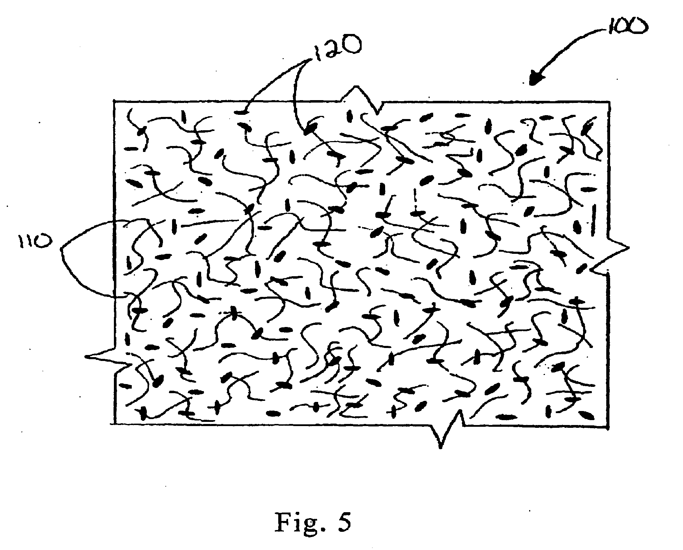

Figure 5 depicts a schematic plan view of an embodiment of a fibrous structure of the present invention in which the synthetic fibers and natural fibers are distributed randomly throughout the fibrous structure.
Figure 6 is a schematic top view of a strike through plate which may be used to measure Liquid Strike Through of a substrate.
Figure 7 is a section view along 9-9 of the strike through place of Figure 6.
Figure 8 is a sectional perspective view along 10-10 of the strike through plate of Figure 6.

## DETAILED DESCRIPTION OF THE INVENTION

As used herein, the following terms have the following meanings.

"Basis weight" refers to the weight (measured in grams) of a unit area (typically measured in square meters) of the fibrous structure, which unit area is taken in the plane of the fibrous structure. The size and shape of the unit area from which the basis weight is measured is dependent upon the relative and absolute sizes and shapes of the regions having different basis weights.

"Binder" and/or "Binder material" refers to the various wet and dry strength resins and retention aid resins known in the paper making art.

"Coarseness" refers to the weight per unit length of fiber expressed as milligrams per 100m, as set forth in TAPPI Method T 234 cm-02.

"Hydrophilizing agent" may be broadly disclosed as comprising oligomeric or polymeric "Backbones" to which are appended hydrophilic substituents. "Oligomeric" herein refers to a polymer molecule with fewer than 10 repeating units such as dimers, trimers, tetramers, etc. "Polymeric" herein refers to a molecule with greater than 10 repeating units. A wide variety of such agents are, as noted above, very well known for use as soil release compounds in the detergency arts. The manufacture of such agents forms no part of this invention. Reference can be made to a series of patents more fully disclosing such compounds, as well as their method of synthesis, as disclosed hereinafter. The present invention employs such compounds, and their equivalents, in the improved nonwoven fibrous structure described herein. Such compounds are usually water-soluble or water-dispersible under the preferred usage conditions herein, e.g., in a fiber slurry comprising an aqueous carrier medium; 20°C - 90°C operating conditions; usage levels of about 0.001% to about 20%, by weight of the fiber weight; weight ratio of hydrophilizing agent : hydrophobic fiber in slurry in the range of from about 0.0001:1 to about 1:1.

"Nonwoven" refers to a fibrous structure made from an assembly of continuous fibers, co-extruded fibers, non-continuous fibers, and combinations thereof, without weaving or knitting, by processes such as spun-bonding, carding, melt-blowing, air-laying, wet-laying, co-form, or other processes known in the art for such purposes. The non-woven structure may comprise one or more layers of such fibrous assemblies, wherein each layer may include continuous fibers, co-extruded fibers, non-continuous fibers, and combinations thereof.

"Unitary fibrous structure" or "fibrous structure" refers to an arrangement comprising a plurality of synthetic fibers that are inter-entangled to form a single-ply sheet product having certain pre-determined microscopic geometric, physical, and aesthetic properties. The fibrous structure may further comprise natural fibers. The synthetic and/or natural fibers may be layered, as known in the art, in the unitary fibrous structure. The fibrous structure may be non-woven. The fibrous structure may be useful as a web for tissue grades of paper (i.e., sanitary tissue products) such as toilet paper, paper towels, napkins, facial tissue, sanitary products such as wipes, and absorbent articles such as diapers, feminine pads and incontinence articles. The fibrous structure of the present invention may be incorporated into an article, such as a single or multi-ply sanitary tissue product. The fibrous structure of the present invention may be layered or may be homogeneous.

## Fibrous Structure

The fibrous structure of the present invention may take a number of different forms. The fibrous structure may comprise 100% synthetic fibers or may be a combination of synthetic fibers and natural fibers. In one embodiment of the present invention, the fibrous structure may include one or more layers of a plurality of synthetic fibers mixed with a plurality of natural fibers. The synthetic fiber/natural fiber mix may be relatively homogeneous in that the different fibers may be dispersed generally randomly throughout the layer. The fiber mix may be structured such that the synthetic fibers and natural fibers may be disposed generally non-randomly. In one embodiment, the fibrous structure may include at least one layer comprising a plurality of natural fibers and at least one adjacent layer comprising a plurality of synthetic fibers. In another embodiment, the fibrous structure may include at least one layer that comprises a plurality of synthetic fibers homogeneously mixed with a plurality of natural fibers and at least one adjacent layer that comprises a plurality of natural fibers. In an alternate embodiment, the fibrous structure may include at least one layer that comprises a plurality of natural fibers and at least one adjacent layer that may comprise a mixture .of a plurality of synthetic fibers and a plurality of natural fibers in which the synthetic fibers and/or natural fibers may be disposed generally non-randomly. Further, one or more of the layers of mixed natural fibers and synthetic fibers may be subjected to manipulation during or after the formation of the fibrous structure to disperse the layer or layers of mixed synthetic and natural fibers in a predetermined pattern or other non-random pattern. Such a pattern may be a repeating pattern.

Examples of natural fibers may include cellulosic natural fibers, such as fibers from hardwood sources, softwood sources, or other non-wood plants. The natural fibers may comprise cellulose, starch and combinations thereof. Non-limiting examples of suitable cellulosic natural fibers include wood pulp, typical northern softwood Kraft, typical southern softwood Kraft, typical CTMP, typical deinked, corn pulp, acacia, eucalyptus, aspen, reed pulp, birch, maple, radiata pine and combinations thereof. Other sources of natural fibers from plants include, but are not limited to, albardine, esparto, wheat, rice, corn, sugar cane, papyrus, jute, reed, sabia, raphia, bamboo, sidal, kenaf, abaca, sunn, rayon, lyocell, cotton, hemp, flax, ramie and combinations thereof. Yet other natural fibers may include fibers from other natural non-plant sources, such as, down, feathers, silk, and combinations thereof. The natural fibers may be treated or otherwise modified mechanically or chemically to provide desired characteristics or may be in a form that is generally similar to the form in which they can be found in nature. Mechanical and/or chemical manipulation of natural fibers does not exclude them from what are considered natural fibers with respect to the development described herein.

The synthetic fibers can be any material, such as, but not limited to, those selected from the group consisting of polyesters, polypropylenes, polyethylenes, polyethers, polyamides, polyhydroxyalkanoates, polysaccharides, and combinations thereof. The synthetic fibers may comprise a polymer. The polymer may be any material such as, but not limited to, those materials selected from the group consisting of polyesters, polyamides, polyhydrdxyalkanoates, polysaccharides, and combinations thereof. More specifically, the material of the polymer segment may be selected from the group consisting of poly(ethylene terephthalate), poly(butylene terephthalate), poly(1,4-cyclohexylenedimethylene terephthalate), isophthalic acid copolymers (e.g., terephthalate cyclohexylene-dimethylene isophthalate copolymer), ethylene glycol copolymers (e.g., ethylene terephthalate cyclohexylene-dimethylene copolymer), polycaprolactone, poly (hydroxyl ether ester), poly(hydroxyl ether amide), polyesteramide, poly(lactic acid), polyhydroxybutyrate, and combinations thereof. The polymer may comprise a segment, such as a polymer segment that may be complementary to a hydrophilizing agent and/or a segment thereof. The portion of the polymer segment that may be complementary to a hydrophilizing agent may facilitate association between the synthetic fiber and the hydrophilizing agent. The complementary segment may comprise a polyester segment. The polyester segment may further comprise a polyethylene terephthalate segment. The complementary segment of the polymer may be located on the surface of the synthetic fiber. Such may be the situation wherein the synthetic fiber may be a bicomponent fiber comprising a core and an outer surface.

Further, the synthetic fibers can be a single component (i.e., single synthetic material or mixture makes up entire fiber), bi-component (i.e., the fiber is divided into regions, the regions including two or more different synthetic materials or mixtures thereof and may include co-extruded fibers) and combinations thereof. It is also possible to use bicomponent fibers, or simply bicomponent or sheath polymers. These bicomponent fibers can be used as a component fiber of the structure, and/or they may be present to act as a binder for the other fibers present in the nonwoven material. Any or all of the synthetic fibers may be treated before, during, or after the process of the present invention to change any desired properties of the fibers. For example, in certain embodiments, it may be desirable to treat the synthetic fibers before or during the papermaking process to make them more hydrophilic, more wettable, etc.

In certain embodiments of the present invention, it may be desirable to have particular combinations of fibers to provide desired characteristics. For example, it may be desirable to have fibers of certain lengths, widths, coarseness or other characteristics combined in certain layers or separate from each other. The fibers may have an average fiber length of greater than about 0.20 mm. The fibers may have an average fiber length of from about 0.20 mm to about 10.0 mm. The fibers may have an average fiber width of greater than about 5 micrometers. The fibers may have an average fiber width of from about 5 micrometers to about 50 micrometers. The fibers may have a coarseness of greater than about 5mg/100m. The fibers may have a coarseness of from about 5mg/100m to about 75mg/100 m. Individually, the fibers may have certain desired characteristics.

The fibrous structure may further comprise a binder material. The fibrous structure may comprise from about 0.01% to about 1%, 3%, or 5% by weight of a binder material selected from the group consisting of permanent wet strength resins, temporary wet strength resins, dry strength resins, retention aid resins and combinations thereof.

If permanent wet strength is desired, the binder material may be selected from the group consisting of polyamide-epichlorohydrin, polyacrylamides, styrene-butadiene latexes, insolubilized polyvinyl alcohol, ureaformaldehyde, polyethyleneimine, chitosan polymers and combinations thereof.

If temporary wet strength is desired, the binder material may be selected from the groups of starch-based temporary wet strength resins consisting of cationic dialdehyde starch-based resin, dialdehyde starch and combinations thereof. The resin described in US Patent No. 4,981,557 may also be used.

If dry strength is desired, the binder material may be selected from the group consisting of polyacrylamide, starch, polyvinyl alcohol, guar or locust bean gums, polyacrylate latexes, carboxymethyl cellulose and combinations thereof.

A latex binder material may also be utilized. Such a latex binder may have a glass transition temperature from about 0°C, -10°C, or -20°C to about -40°C, -60°C, or -80°C. Examples of latex binders that may be used include, but are not limited to, polymers and copolymers of acrylate esters, referred to generally as acrylic polymers, vinyl acetate-ethylene copolymers, styrene-butadiene copolymers, vinyl chloride polymers, vinylidene chloride polymers, vinyl chloride-vinylidene chloride copolymers, acrylo-nitrile copolymers, acrylic-ethylene copolymers and combinations thereof. The water emulsions of these latex binders usually contain surfactants. These surfactants may be modified during drying and curing so that they become incapable of rewetting.

Methods of application of the binder material may include aqueous emulsion, wet end addition, spraying and printing. At least an effective amount of binder material may be applied to the fibrous structure. Between about 0.01% and about 1.0%, 3.0% or 5.0% may be retained on the fibrous structure, calculated on a dry fiber weight basis. The binder material may be applied to the fibrous structure in an intermittent pattern generally covering less than about 50% of the surface area of the structure. The binder material may also be applied to the fibrous structure in a pattern to generally cover greater than about 50% of the fibrous structure. The binder material may be disposed on the fibrous structure in a random distribution. Alternatively, the binder material may be disposed on the fibrous structure in a non-random repeating pattern.

Additional information relating to the fibrous structure may be found in U.S. Patent Publication Nos. 2004/0154768 and 2004/0157524, US Patent Nos. 4,588,457; 5,397,435; and 5,405,501.

A variety of products can be made using the fibrous structure of the present invention. The resultant products may be disposable. The resultant products may find use in filters for air, oil and water; vacuum cleaner filters; furnace filters; face masks; coffee filters, tea or coffee bags; thermal insulation materials and sound insulation materials; nonwovens for absorbent articles such as diapers, feminine pads, and incontinence articles; biodegradable textile fabrics for improved moisture absorption and softness of wear such as microfiber or breathable fabrics; an electrostatically charged, structured web for collecting and removing dust; reinforcements and webs for hard grades of paper, such as wrapping paper, writing paper, newsprint, corrugated paper board, and webs for tissue grades of paper such as toilet paper, paper towel, napkins, and facial tissue; medical uses such as surgical drapes, wound dressing, bandages, and dermal patches. The fibrous structure may also include odor absorbents, termite repellents, insecticides, rodenticides, and the like, for specific uses. The resultant product may absorb water and oil and may find use in oil or water spill clean-up, for controlled water retention and release for agricultural or horticultural applications.

Absorbent Article

The fibrous structure, as described above, may be utilized to form a component of an absorbent article. "Absorbent article" refers to devices which may absorb and may contain bodily exudates, and, more specifically, refers to devices which may be placed against or in proximity to the body of the wearer to absorb and contain the various exudates discharged from the body. This may include, but is not limited to, urine, menses and vaginal discharges, sweat and feces. Examples of illustrative disposable absorbent articles include but are not limited to, diapers, adult incontinence products, training pants, feminine hygiene pads, panty liners and the like.

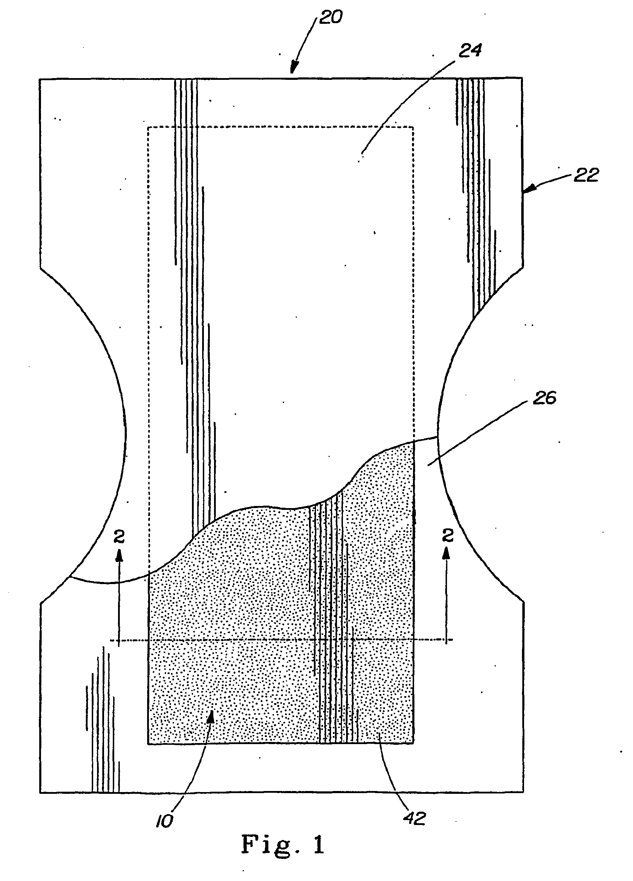

Figure 1 is a plan view of a disposable absorbent article, specifically a diaper 20. The diaper 20 is shown in its flat out, uncontracted state (i.e., without elastic induced contraction). Portions of the structure are cut away to show the underlying structure of the diaper 20, including the absorbent core 10. The portion of the diaper 20 that contacts a wearer is facing the viewer. The chassis 22 of the diaper 20 in Figure 1 may comprise the main body of the diaper 20. The chassis 22 may comprise an outer covering including a liquid pervious topsheet 24 and/or a liquid impervious backsheet 26. The chassis 22 may also include most or all of the absorbent core 10 encased between the topsheet 24 and the backsheet 26.

The chassis 22 may comprise the main structure of the diaper 20 with other features added to form the composite diaper structure. The topsheet 24, backsheet 26, and absorbent core 10 may include many different materials and may be assembled in a variety of well known configurations, exemplary diaper materials and configurations are described generally in US Patent Nos. 3,860,003; 5,151,029; 5,221,274; 5,569,234 and 6,004,306.

Any topsheet compatible with the present invention which is known in the art can be used in the present invention. A suitable material for a topsheet may be manufactured from a wide range of materials, such as porous foams, reticulated foams, apertured plastic films, or woven or nonwoven materials of natural fibers (e.g., wood or cotton fibers), synthetic fibers (e.g., polyester or polypropylene fibers), or a combination of natural and synthetic fibers. As an example, a material suitable for use in a topsheet comprises a web of staple-length polypropylene fibers is manufactured by Veratec, Inc., a Division of International Paper Company, of Walpole, MA under the designation P-8.

Some examples of suitable topsheets are described further in U.S. Patent Nos. 3,929,135; 4,324,246; 4,342,314; 4,463,045; 5,006,394; 4,609,518; and 4,629,643. Any portion of the topsheet may be coated with a lotion as is known in the art. Examples of suitable lotions include those described in U.S. Patent Nos. 5,607,760; 5,609,587; 5,635,191; 5,643,588; 5,968,025; 6,716,441; and PCT Publication No. WO 95/24173.

The topsheet 24 in Figure 1 may be fully or partially elasticized or may be foreshortened to provide a void space between the topsheet 24 and the absorbent core 10. Exemplary structures including elasticized or foreshortened topsheets are described in more detail in U.S. Pat. Nos. 4,892,536; 4,990,147; 5,037,416 and 5,269,775.

The backsheet 26 in Figure 1 may generally be the portion of the diaper 20 positioned with the absorbent core 10 between the backsheet 26 and the topsheet 24. The backsheet 26 may be joined with the topsheet 24. The backsheet 26 may prevent the exudates absorbed by the absorbent core 10 and contained within the diaper 20 from soiling other external articles that may contact the diaper 20, such as bed sheets and undergarments. The backsheet 26 may be substantially impervious to liquids (e.g., urine) and may comprise a laminate of a nonwoven and a thin plastic film such as a thermoplastic film having a thickness of between about 0.012 mm (0.5 mil) to about 0.051 mm (2.0 mils). Suitable backsheet films may include those manufactured by Tredegar Industries Inc. of Terre Haute, IN and sold under the trade names X15306, X10962, and X10964. Other suitable backsheet materials may include breathable materials that permit vapors to escape from the diaper 20 while still preventing exudates from passing through the backsheet 26. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, TX, under the designation EXXAIRE. Suitable breathable composite materials comprising polymer blends are available from Clopay Corporation, Cincinnati, OH under the name HYTREL blend P18-3097.

Diapers according to the present invention may be provided with a re-closable fastening system which may include at least one fastening member and at least one stored landing zone. In order to keep the diaper in place about the wearer, a portion of the first waist region may be attached by a fastening member to a portion of the second waist region, thereby forming leg opening(s) and a waist. Alternatively, the diaper may be provided in the form of pant-type diapers. The diaper may also include such other features as are known in the art including waist cap features, elastics and the like to provide better fit, containment and aesthetic characteristics. The chassis may further include side panels, elasticized leg cuffs, and an elastic waist feature, wherein the leg cuffs and the elastic waist feature each typically comprise elastic members. One end portion of the diaper may be configured as a first waist region of the diaper. The opposite end portion may be configured as a second waist region of the diaper. An intermediate portion of the diaper may be configured as a crotch region, which may extend longitudinally between the first and second waist regions. The waist regions may include elastic elements such that they gather about the waist of the wearer to provide improved fit and containment. The crotch region may be that portion of the diaper which, when the diaper is worn, is generally positioned between the wearer's legs. Additional details regarding the waist features and cuffs may be found in US Patent Nos. 3,860,003; 4,515,595; 4,695,278; 4,909,803; 5,151,092; and 5,221,274.

The diaper 20 may include front ears and back ears. The front and/or back ears may be unitary elements of the diaper 20 (*i.e.*, they are not separately manipulative elements secured to the diaper 20, but rather are formed from and are extensions of one or more of the various layers of the diaper). In certain embodiments, the front and/or back ears may be discrete elements that are joined to the chassis 22, as shown in FIG. 1. In other embodiments, the front and/or back ears may comprise a discrete element joined to the chassis 22 with the chassis 22 having a layer, element, or substrate that extends over the front and/or back ear. The front ears and back ears may be extensible, inextensible, elastic, or inelastic. The front and/or back ears may also be subjected to activation, mechanical activation, ring rolling, etc. as described in U.S. Patent Nos. 5,167,897; 5,143,679; 5,156,793; 5,705,013; 5,683,533; and 5,580,411. The term "activation" or "activating" refers herein to the process of making a nonwoven, or an elastic and nonwoven laminate more extensible.

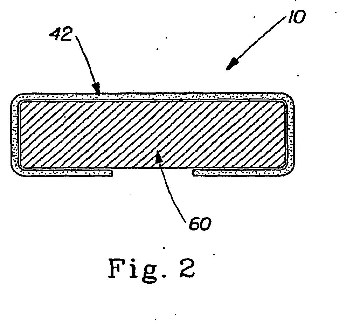

The absorbent article may comprise an absorbent core that may be primarily responsible for fluid handling properties of the article, including acquiring, transporting, distributing and storing body fluids. As such, the absorbent core typically does not include the topsheet or backsheet of the absorbent article. The absorbent core 10 in Figure 1 generally is disposed between the topsheet 24 and the backsheet 26. The absorbent core 10 may comprise a core cover 42 and a storage layer 60 as shown in Figure 2. The storage layer 60 may comprise any absorbent material that is generally compressible, conformable, non-irritating to the wearer's skin, and capable of absorbing and retaining liquids such as urine and other certain body exudates. The storage layer 60 may comprise a wide variety of liquid-absorbent materials commonly used in disposable diapers and other absorbent articles such as comminuted wood pulp, which is generally referred to as air felt or fluff. Examples of other suitable absorbent materials include creped cellulose wadding; melt blown polymers, including co-form; chemically stiffened, modified or cross-linked cellulosic fibers as described in US Patent No. 5,137,537; tissue, including tissue wraps and tissue laminates, absorbent foams, absorbent sponges, superabsorbent polymers (such as superabsorbent fibers) such as those described in US Patent No. 5,599,335; absorbent gelling materials, or any other known absorbent material or combinations of materials. Examples of some combinations of suitable absorbent materials are fluff with absorbent gelling materials and/or superabsorbent polymers, and absorbent gelling materials and superabsorbent fibers etc. In one optional embodiment the storage layer is air felt free, that is, it contains no air felt. The storage layer may further comprise minor amounts (typically less than 10%) of non-liquid absorbent materials, such as adhesives, waxes, oils and the like.

The storage layer of the absorbent core may comprise absorbent polymer material. The absorbent polymer material may also be mixed with absorbent fibrous material, such as airfelt material, which can provide a matrix for immobilization of the super-absorbent polymer material. However, a relatively low amount of fibrous cellulose material may be used, such as less than about 40%, 20% or 10% weight of cellulose fibrous material as compared to the weight of absorbent polymer material. Substantially airfelt free cores may be useful as well.

Optionally, the storage layer of the absorbent core can also comprise an absorbent fibrous material, for example cellulose fibers. This fibrous material can be pre-mixed with the absorbent polymeric material and be laid down in one process step or it can alternatively be laid-down in separate process steps.

Additionally, suitable absorbent cores may contain reduced amounts of cellulosic airfelt material. For instance, such cores may comprise less than about 40%, 30%, 20%, 10%, 5%, or even about 1%. Such a core comprises primarily absorbent gelling material in amounts of at least about 60%, 70%, 80%, 85%, 90%, 95% or even about 100%, where the remainder of the core comprises a microfiber glue (if applicable). Such cores, microfiber glues, and absorbent gelling materials are described in U.S. Patent Nos. 5,599,335; 5,562,646; 5,669,894; 6,790,798; and U.S. Patent Publications 2004/0158212A1 and 2004/0097895A1; and U.S. Application Ser. Nos. 10/758,375 and 10/758,138, both filed on January 15, 2004.

In further embodiments, the articles of the present invention may further comprise a wetness sensation member. This member may be disposed in various locations within the article. For instance, the wetness sensation member may be disposed on the topsheet. The member may comprise a permeable layer and an impermeable layer, wherein urine passes through the permeable layer and not through the impermeable layer such that a wearer is made aware of the fact that urination has occurred as a result of the "wet" feeling. Suitable members are detailed in U.S. Patent No. 6,627,786.

An absorbent article according to the present invention may comprise a relatively narrow crotch width, which may increase the wearing comfort. An absorbent article of the present invention may comprise a crotch width of less than about 100 mm, 90 mm, 80 mm, 70 mm, 60 mm or even less than about 50 mm. Hence, an absorbent core according to the present invention may have a crotch width as measured along a transversal line which is positioned at equal distance to the front edge and the rear edge of the core which may be less than about 100 mm, 90 mm, 80 mm, 70 mm, 60 mm or even less than about 50 mm. It has been found that for most absorbent articles the liquid discharge occurs predominately in the front half. The front half of the absorbent core should therefore comprise most of the absorbent capacity of the core. The front half of said absorbent core may comprise more than about 60% of the absorbent capacity, or more than about 65%, 70%, 75%, 80%, 85%, or 90%.

These materials may be combined to provide an absorbent core in the form of one or more layers that may include fluid handling layers such as an acquisition layer, such as described in published WO 98/22279; distribution layer, such as one comprising chemically stiffened, modified or cross-linked cellulosic fibers; and storage layers such as one comprising superabsorbent polymers. The absorbent core may also include layers that may stabilize other core components. Such layers include a core cover, that may overlie a storage layer and underlie any other core components if such components are present, and a dusting layer, that may underlie a storage layer. Suitable materials for such layers may include spunbond/meltblown/spunbond nonwovens having a basis weight between about 10 and about 15 g/m$^2$ (the meltblown comprises less than about 5 g/m$^2$). The fibrous structure described herein is also suitable for use in such layers.

The acquisition layer, distribution layer, storage layer, core cover, and dusting layer may generally be known as wrap sheets. Nonwoven wrap sheets may be fibrous structures, such as the fibrous structure described herein, which may have the primary functionality of containing materials of the absorbent core therein without detrimentally impacting on the fluid handling properties of the absorbent core, even for subsequent gushes. The containment functionality may be achieved by fibrous structures having small mean pore sizes, such as less than 30 $\mu$m when measured by the Coulter Porometer Mean Flow Pore size and Pore size distribution test in accordance with ASTM Test Method F316-86.

The wrap sheets may be permeable to aqueous liquids, such as by being porous like fibrous webs or perforated film materials. The wrap sheet may completely envelope the absorbent core. Alternatively, the wrap sheet need not completely envelope the absorbent core. The wrap sheet may cover the top surface of the absorbent core and may then be tacked down next to the core, such that the side surface may be, but not necessarily have to be, covered by the wrap sheet. In yet another embodiment, the wrap sheet may cover the top surface of the absorbent core as well as two side surfaces by being folded around these surfaces to partly or fully cover the bottom surface.

The wrapping of the absorbent core can also be achieved by more than one wrap sheet, or by one wrap sheet with different properties in different regions thereof. For example, the surface parts of the absorbent core which are not in the fluid flow path, can have not, or non-permanent fluid hydrophilicity. Or, a different wrap material can be used in such regions, or the absorbent core materials can there be contained by other elements such as conventional tissue materials, but also impermeable sheets, which may at the same time have other functionalities.

The liquid strike through time is a measure of a certain hydrophilicity level of fibrous structures. The value may be measured using the test method described herein.

Hydrophilizing Agent

Figure 1 is illustrative, but by no means limiting, of an artist's conception at the molecular level of a hydrophilizing agent 1 having a dimeric "backbone," a complementary segment 3, and hydrophilic substituents 4 associated with a complementary segment of a synthetic fiber 2, wherein n may be from about 1 to about 15.

While not intending to be limited by theory, it is surmised that the hydrophilizing agent becomes associated with one or more surfaces of the hydrophobic synthetic fiber. The association between the hydrophilizing agent and the synthetic fiber may be a durable association. The association of the hydrophilizing agent with the synthetic fibers may provide for the synthetic fibers to exhibit hydrophilic characteristics as opposed to the hydrophobic characteristics displayed by the synthetic fibers alone. It is further surmised that the hydrophobicity of synthetic fibers alone may generally cause the synthetic fibers to clump together during the webmaking process or within a fibrous structure. Whatever the reason, it has now been found that the association of a hydrophilizing agent with the synthetic fibers may provide for the dispersion of the synthetic fibers in a fibrous structure. For example, during a wet laid papermaking process, there may be a dispersion of the synthetic fibers in a fluid carrier which may then promote the dispersion of the synthetic fibers in the fibrous structure. Natural fibers may optionally be present in the dispersion as the natural fibers may not interfere with the association of the hydrophilizing agent to the synthetic fibers. The hydrophilizing agent may' associate with the natural fibers; however, this association will not prevent the hydrophilizing agent from associating with the synthetic fibers.

Hydrophilizing agents can include a variety of charged anionic or cationic species as well as noncharged monomer units. The anionic and cationic polymers may enhance both the deposition and the wettability of the synthetic fibers. Hydrophilizing agents comprising cationic functionalities are disclosed in US 4,956,447. The structure of the hydrophilizing agents may be linear, branched or even star-shaped. Structures and charge distributions may be tailored for application to different fiber or textile types.

The hydrophilizing agent may associate with the synthetic fibers by a correspondence between the hydrophilizing agent and the surface characteristics of the synthetic fibers. This correspondence may be based on physical characteristics of the synthetic fibers and hydrophilizing agent. Such physical characteristics may include, but are not limited to, degree of crystallinity and molecular weight. Correspondence between the physical characteristics of the hydrophilizing agents and the synthetic fibers may aid in the durability of the association formed between the hydrophilizing agents and the synthetic fibers. It has been found that an association based upon physical characteristics may be durable wherein the hydrophilizing agent may not wash off from the synthetic fibers. As such, the hydrophilizing agents of the present invention may be distinguished from typical surfactants. The bond between the synthetic fibers and the hydrophilizing agent may be durable. The synthetic fibers may exhibit a durable wettability. The synthetic fibers may exhibit a mean contact angle of less than about 72°. The synthetic fibers may exhibit a mean contact angle of less than about 72° and after a 10 minute water wash the mean contact angle of the synthetic fibers may remain below about 72°. The synthetic fibers may exhibit a mean contact angle following a 10 minute water wash of less than about 66°, 63°, 60°, 55° or 50°. The synthetic fibers exhibiting such mean contact angles may be associated with a hydrophilizing agent. The bond between the synthetic fibers and the hydrophilizing agent may be durable and the hydrophilizing agent may not be washed off the synthetic fibers after a single insult of fluid. A surfactant, on the other hand, is unable to form such a durable bond and may be washed off the synthetic fibers upon a single insult of fluid. Furthermore, a fibrous structure comprising synthetic fibers and a hydrophilizing agent may demonstrate a sustainable wettability, as detailed herein, whereas a fibrous structure comprising synthetic fibers and a surfactant may not exhibit a sustainable wettability. A more permanent association may be made between the hydrophilizing agent and the synthetic fibers by heating the combination of the hydrophilizing agent and the synthetic fibers above the melting temperature of the hydrophilizing agent.

Hydrophilizing agents may comprise greater than about 3 ppm of a hydrophilizing agent/synthetic fiber and/or natural fiber combination. Hydrophilizing agents may generally comprise from about 10, 20, 30 or 40 ppm to about 50, 60, 80 or 100 ppm of a hydrophilizing agent/synthetic fiber and/or natural fibers combination. The compositions herein may comprise greater than about 0.001% of a hydrophilizing agent. The compositions herein may comprise from about 0.001 % to about 2%, 5%, 10% or 20% of a hydrophilizing agent.

The hydrophilizing agent may comprise a segment that may be complementary to the polymer of the synthetic fibers. The complementary segment may comprise a polyester segment. The polyester segment may comprise a polyethylene terephthalate segment. The hydrophilizing agent may be oligomeric or polymeric. The hydrophilizing agent may be a copolymer of ethoxylate siloxane. The hydrophilizing agent may be soil release agent. Such a hydrophilizing agent may be a polymer. Polymeric hydrophilizing agents useful in the present invention may include, but are not limited to materials selected from the group consisting of polyester, poly(ethoxylate), polyethylene oxide, polyoxyethylene, polyethylene glycol, polypropylene glycol, terephthalate, polypropylene oxide, polyethylene terephthalate, polyoxyethylene terephthalate, ethoxylate siloxane and combinations thereof. Polyesters of terephthalic and other aromatic dicarboxylic acids having soil release properties such as polyethylene terephthalate/polyoxyethylene terephthalate and polyethylene terephthalate/polyethylene glycol polymers, among other polyester polymers, may be utilized as the hydrophilizing agent in the fibrous structure. As noted above, a wide variety of hydrophilizing agents, also known as SRP's, SRA's, and SRE's, are well-recognized materials in the detergency arts, and many are available commercially or by synthesis schemes disclosed in multiple patents of The Procter & Gamble Company and various manufacturers.

Higher molecular weight (e.g., 40,000 to 50,000 M.W.) polyesters containing random or block ethylene terephthalate/polyethylene glycol (PEG) terephthalate units have been used as soil release agents in laundry cleaning compositions. See U.S. Patent Nos. 3,893,929; 3,959,230; and 3,962,152. Sulfonated linear terephthalate ester oligomers are disclosed in U.S. 4,968,451. Nonionic end-capped 1,2-propylene/polyoxyethylene terephthalate polyesters are disclosed in U.S. 4,711,730 and nonionic-capped block polyester oligomeric compounds are disclosed in U.S. 4,702,857. Partly- and fully- anionic-end-capped oligomeric esters are disclosed further in U.S. 4,721,580 and anionic, especially sulfoaroyl, end-capped terephthalate esters are disclosed in U.S. 4,877,896 and U.S. 5,415,807.

U.S. 4,427,557 discloses low molecular weight copolyesters (M.W. 2,000 to 10,000) which can be used in aqueous dispersions to impart soil release properties to polyester fibers. The copolyesters are formed by the reaction of ethylene glycol, a PEG having an average molecular weight of 200 to 1000, an aromatic dicarboxylic acid (e.g., dimethyl terephthalate), and a sulfonated aromatic dicarboxylic acid (e.g., dimethyl 5-sulfoisophthalate). The PEG can be replaced in part with monoalkylethers of PEG such as the methyl, ethyl and butyl ethers.

A hydrophilizing agent may be a copolymer having blocks of terephthalate and polyethylene oxide. More specifically, these polymers may comprise repeating units of ethylene and/or propylene terephthalate and polyethylene oxide terephthalate at a molar ratio of ethylene terephthalate units to polyethylene oxide terephthalate units of from about 25:75 to about 35:65, said polyethylene oxide terephthalate containing polyethylene oxide blocks having molecular weights of from about 300 to about 2000. The molecular weight of this polymeric hydrophilizing agent may be in the range of from about 5,000 to about 55,000.

Another polymeric hydrophilizing agent may be a crystallizable polyester with repeat units of ethylene terephthalate units comprising from about 10% to about 15% by weight of ethylene terephthalate units together with from about 10% to about 50% by weight of polyoxyethylene terephthalate units, derived from a polyoxyethylene glycol of average molecular weight of from about 300 to about 6,000, and the molar ratio of ethylene terephthalate units to polyoxyethylene terephthalate units in the crystallizable polymeric compound may be between 2:1 and 6:1. Examples of this polymer include the commercially available materials ZELCON® 4780 (from DuPont) and MILEASE® T (from ICI).

In another embodiment, the poly(ethoxylate) regions may be tailored to have from about 1 to about 9, 12, or 15 ethoxylated groups and any other number of ethoxylated groups within the range of from about 1 to about 15. The number of poly(ethoxylated) regions may be tailored to enhance the wettability of the synthetic fibers. Wettability of the synthetic fibers may be increased as the number of ethoxylated groups increases in the poly(ethoxylate) regions. Optionally, additional copolymers such as, but not limited to, polyethylene glycol and polypropylene glycol, may be used to control the crystallinity of the hydrophilizing agents.

In an alternative embodiment, the hydrophilizing agents provided by the invention may be illustrated by one comprising from about 25% to about 100% by weight of an ester having the empirical formula $(CAP)_x(EG/PG)_{y'}(DEG)_{y''}$, $(PEG)_{y'''}(T)_z(SIP)_q$; wherein (CAP) represents the sodium salt form of said end-capping units i); (EG/PG) represents said oxyethyleneoxy and oxy-1,2-propyleneoxy units ii); (DEG) represents said di(oxyethylene)oxy units iii); (PEG) represents said poly(oxyethylene)oxy units iv); (T) represents said terephthaloyl units v); (SIP) represents the sodium salt form of 5-sulfoisophthaloyl units vi); x is from about 1 to 2; y' is from about 0.5 to about 66; y" is from 0 to about 50; y''' is from 0 to about 50; y'+y"+y''' totals from about 0.5 to about 66; z is from about 1.5 to about 40; and q is from about 0.05 to about 26; wherein x, y', y", y''', z and q represent the average number of moles of the corresponding units per mole of said ester. Hydrophilizing agents may be those wherein at least about 50% by weight of said ester has a molecular weight ranging from about 500 to about 5,000.

In one embodiment, the hydrophilizing agents may have oxyethyleneoxy:oxy-1,2-propyleneoxy mole ratio ranges from about 0.5:1 to about 10:1; x is about 2, y' is from about 2 to about 27, z is from about 2 to about 20, and q is about 0.4 to about 8. In another embodiment, x is about 2, y' is about 5, z is about 5, and q is about 1.

The hydrophilizing agents may associate with the synthetic fiber surface during the process of re-pulping the fibers. The synthetic fibers may also be provided with a finishing coat of the hydrophilizing agent prior to re-pulping the fibers. Additionally, the hydrophilizing agent may associate with the synthetic fibers as a melt-additive prior to extrusion of the synthetic fibers.

Additional information relating to hydrophilizing agents may be found in U.S. Patent Nos. 4,702,857; 4,861,512; 5,574,179 and 5,843,878.


Method of Making Fibrous Structure


Generally, the process of the present invention for making a unitary fibrous structure may be described in terms of forming a web having a plurality of synthetic fibers disposed in a generally random pattern throughout the fibrous structure. A plurality of natural fibers may also be disposed in a generally random pattern throughout the fibrous structure. In another embodiment, a portion of the synthetic fibers may be redistributed in a non-random repeating pattern. Layered deposition of the fibers, synthetic and natural, is also contemplated by the present invention.

In a typical wet-laid process, the plurality of fibers may be suspended in a fluid carrier. This may also be known as "re-pulping" the fibers. Synthetic fibers may be re-pulped separately from or in combination with natural fibers. In another embodiment, a plurality of synthetic fibers and a plurality of natural fibers may both be added to a re-pulper. A hydrophilizing agent may then be added to the re-pulper to associate with the synthetic fibers. In yet another embodiment, a plurality of synthetic fibers may be added to a re-pulper and mixed with a hydrophilizing agent. This combination may then be mixed with a plurality of natural fibers. As an alternative, the synthetic fibers may associate with a hydrophilizing agent by providing the synthetic fibers with a finishing coat containing a hydrophilizing agent prior to being re-pulped. The synthetic fibers may then be re-pulped and combined with natural fibers. Alternatively, the hydrophilizing agent may associate with the synthetic fibers as a melt-additive prior to extrusion of the fibers.

In yet another embodiment, the process may be air-laid in which a plurality of synthetic fibers associated with a hydrophilizing agent are placed directly into the papermaking machinery. In such an embodiment, a plurality of natural fibers may also be placed directly into the papermaking machinery. In addition to wet-laid and air-laid, other methods may include, but are not limited to, melt-blown, spun-bond, carded, co-form, adhesive bonding, needle punched, hydroentangled, lamination or other processes known in the art for such purposes. Combinations of the methods can also be used.

The resulting fibrous structure may comprise natural and synthetic fibers dispersed generally randomly throughout the layer. Alternatively, the natural and synthetic fibers may be more structured such that the synthetic fibers and natural fibers may be disposed generally non-randomly. In one embodiment, the fibrous structure may include at least one layer comprising a plurality of natural fibers and at least one adjacent layer comprising a plurality of synthetic fibers. In another embodiment, the fibrous structure may include at least one layer that comprises a plurality of synthetic fibers homogeneously mixed with natural fibers and at least one adjacent layer that comprises a plurality of natural fibers. In an alternate embodiment, the fibrous structure may include at least one layer that comprises a plurality of natural fibers and at least one adjacent layer that comprises a mixture of a plurality of synthetic fibers and a plurality of natural fibers in which the synthetic fibers and/or natural fibers may be disposed generally non-randomly. Further, one or more of the layers of mixed natural fibers and synthetic fibers may be redistributed in a predetermined pattern or other non-random pattern.

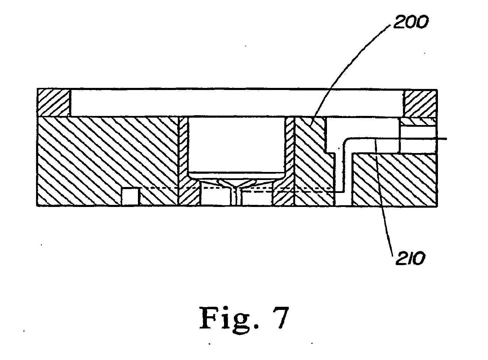

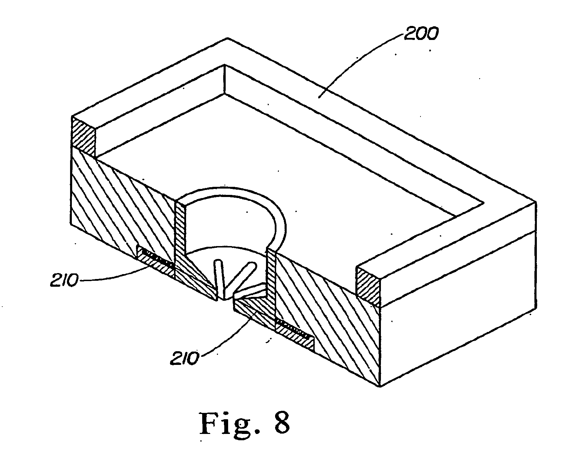

Figure 7 schematically shows one embodiment of the fibrous structure 100 wherein the natural fibers 110 are randomly distributed throughout the structure, and the synthetic fibers 120 are redistributed in a non-random repeating pattern. Figure 8 illustrates a fibrous structure 100 that may comprise a plurality of natural fibers 110 and a plurality of synthetic fibers 120 randomly distributed throughout the fibrous structure.

The following illustrates the practice of the invention, but is not intended to be limiting thereof.

Example 1:

Four different handsheets using Northern Softwood Kraft and CoPET/PET (isophthalic acid copolymers) fibers with or without different hydrophilizing agents are prepared and tested for their impact on Horizontal Absorptive Capacity (H.A.C.) as determined by the Horizontal Full Sheet (HFS) test method described below.

All values below are an average of four separate handsheets.

As shown in the following Table, synthetic fiber addition has a negative impact (~8% loss) on Horizontal Absorptive Capacity (H.A.C.). Addition of hydrophilizing agents makes the synthetic fibers hydrophilic enough to recover the loss in absorptive capacity.

|  | Basis Weight, g/m$^2$ | H.A.C. g/g | H.A.C. Ratio |
|---|---|---|---|
| Sample A (Base) | 26.7 | 11.55 | 1.00 |
| Sample B | 28.3 | 10.60 | 0.92 |
| Sample C | 27.2 | 11.77 | 1.02 |
| Sample D | 27.6 | 11.68 | 1.01 |

Sample A    100% Northern Softwood Kraft (Control sample with cellulosic fibers only)
Sample B    About 70% Northern Softwood Kraft and about 30% CoPET/PET
Sample C    About 70% Northern Softwood Kraft and about 30% CoPET/PET and about 40 ppm TexCare™ SRN-240
Sample D    About 70% Northern Softwood Kraft and about 30% CoPET/PET and about 50 ppm TexCare™ SRN-100

CoPET/PET fibers are commercially available from Fiber Innovation Technology, Inc., Johnson City, TN. The CoPET/PET fibers as used in this example are designated as T-235 by Fiber Innovation Technology. TexCare SRN-100 and TexCare SRN-240 are commercially available from Clairant GmBH, Division Functional Chemicals, Frankfurt am Main.

H.A.C. Ratio = H.A.C. of the Sample/H.A.C. of the Base Sample A

For this Example, the HFS procedure is modified. Four inch (10.2 cm) by 4 inch (10.2 cm) paper samples are used rather than 11 inch (27.9 cm) by 11 inch (27.9 cm) samples as described in the procedure.

Example 2:

A pilot scale Fourdrinier papermaking machine is used in the present example. A 3%, by weight, aqueous slurry of Northern Softwood Kraft (NSK) is made up in a conventional re-pulper. The NSK slurry is refined gently and a 2% solution of a permanent wet strength resin (i.e., Kymene 557LX which is marketed by Hercules Inc., Wilmington, Del.) is added to the NSK stock pipe at a rate of 1%, by weight of the dry fibers. The adsorption of Kymene 557LX to NSK is enhanced by an in-line mixer. A 1% solution of Carboxy Methyl Cellulose (CMC) is added after the in-line mixer at a rate of 0.2%, by weight of the dry fibers, to enhance the dry strength of the fibrous substrate. A 3%, by weight, aqueous slurry of Eucalyptus fibers is made up in a conventional re-pulper.

The NSK slurry and the Eucalyptus fibers are layered in a head box and deposited onto a Fourdrinier wire as different layers to form an embryonic web. Dewatering occurs through the Foudrinier wire and is assisted by a deflector and vacuum boxes. The Fourdrinier wire is of a 5-shed, satin weave configuration having 84 machine-direction and 76 cross-machine-direction monofilaments per inch, respectively. The wet embryonic web is transferred from the Fourdrinier wire, at a fiber consistency of about 18% at the point of transfer, to a photo-polymer fabric having 150 Linear Idaho cells per square inch, 20 percent knuckle areas and 17 mils of photo-polymer depth. Further de-watering is accomplished by vacuum assisted drainage until the web has a fiber consistency of about 22%. The patterned web is pre-dried by air blow-through to a fiber consistency of about 56% by weight. The web is then adhered to the surface of a Yankee dryer with a sprayed creping adhesive comprising 0.25% aqueous solution of Polyvinyl Alcohol (PVA). The fiber consistency is increased to an estimated 96% before dry creping the web with a scalpel blade. The scalpel blade has a bevel angle of about 25 degrees and is positioned with respect to the Yankee dryer to provide an impact angle of about 81 degrees; the Yankee dryer is operated at about 600 fpm (feet per minute) (about 183 meters per minute). The dry web is formed into roll at a speed of 560 fpm (171 meters per minutes).

Two plies of the web are formed into paper towel products by embossing and laminating them together using PVA adhesive. The paper towel has about 40 g/m$^2$ basis weight and contains 70% by weight Northern Softwood Kraft and 30% by weight Eucalyptus furnish. The resulting paper towel has an absorptive capacity of 26.3 gram/gram. The resulting paper towel may also provide a Horizontal Rate Capacity (HRC) value, determined according to the test method described herein. In this example, the HRC value is 0.57 g/sec.

Example 3:

A paper towel is made by a method similar to that of Example 2, but replacing 10% by weight of Eucalyptus by 10% by weight of 6mm in length and about 20 microns in diameter synthetic bicomponent polyester fibers. The polyester fibers as used in this example are available from Fiber Innovation Technology and are designated as T-201. Forty ppm Texcare™ SRN-240 is added to the Eucalyptus-synthetic fiber pulp mixture. The paper towel has about 40 g/m$^2$ basis weight and contains 70% by weight Northern Softwood Kraft in one layer and a mixture of 20% by weight Eucalyptus and 10% by weight of the 6mm long synthetic fibers in the other layer. The resulting paper towel has an absorptive capacity of 26.3 gram/gram. The resulting HRC value for this paper towel is 0.56 g/sec.

Example 4:

A paper towel is made by a method similar to that of Example 2, but replacing 5% by weight of Eucalyptus by 5% by weight of 6mm synthetic bicomponent polyester fibers. The polyester fibers of this example are available from Fiber Innovation Technology and are designated as T-201. Forty ppm TexCare™ SRN-240 is added to the Eucalyptus-synthetic fiber pulp mixture. The paper towel has about 40 g/m$^2$ basis weight and contains 70% by weight Northern Softwood Kraft in one layer and a mixture of 25% by weight Eucalyptus and 5% by weight of the 6mm long synthetic fibers in the other layer. The resulting paper towel has an absorptive capacity of 26.2 gram/gram. The resulting HRC value for this paper towel is 0.57 g/sec.

Example 5:

Two different handsheets using Northern Softwood Kraft and CoPET/PET (isophthalic acid copolymers) fibers with or without different hydrophilizing agents are prepared and tested for their impact on Liquid Handling as determined by the Liquid Strike Through test method described below.

| Sample | Strikethrough[1] (sec) 1st, 3rd, 5th |
|---|---|
| A | 2.5, 3.5, 2.3 ($\pm$0.3, 0.5, 0.2) |
| B | 1.4, 2.2, 1.8 ($\pm$0.2, 0.2, 0.2) |
| [1]GCAS64011333, Lister Model 2005, SN L5877 | |

Sample A    About 30gsm, of about 20% CoPET/PET and about 80% Northern Softwood Kraft and about 40 ppm TexCare™ SRN-240

(continued)

Sample B    About 17gsm, of about 73% Northern softwood Kraft and about 27% CoPET/PET and about 40 ppm Texcare™ SRN-240

CoPET/PET fibers are commercially available from Fiber Innovation Technology, Inc., Johnson City, TN. The CoPET/PET fibers as used in this example are designated as T-201 by Fiber Innovation Technology. TexCare SRN-240 are commercially available from Clairant GmBH, Division Functional Chemicals, Frankfurt am Main.

Example 6:

A disposable article which is a training pant or a diaper as detailed in any one of U.S. Patent Nos. 3,860,003; 4,632,207; 4,695,278; 4,704,115; 4,795,454; 4,900,317; 4,909,803 (Reissued as USRE34920); 5,085,654; 5,492,751; 6,476,288; 6,627,787; 5,507,760; 5,609,587; 5,635,191; 5,643,588; 6,118,041 and SIR H1630 with the exception that the core has been replaced by the one described below. The core that is included in the article of this example consists of a polypropylene dusting layer (13 gsm) that measures about 140 mm x 390 mm. The dusting layer is slot coated longitudinally down the center 97 mm with a suitable adhesive, e.g., HB Fuller 1358 LO, at an add-on amount of about 5 grams along the length. A superabsorbent material, e.g., BASF's ASAP 600Z, is applied via a print roll to the slot coated dusting layer such that a profiled superabsorbent material pattern is deposited onto the dusting layer at an add-on amount of about 9.4 grams along the length of the dusting layer in the form of a rectangle that is 90 mm wide in each of the 4 quarters of its length. A sufficient amount, e.g., of a microfiber glue, e.g., Fuller's NW 1151ZP, is continually applied via spraying onto the superabsorbent material for immobilization of the material. The material of any one of Examples 1 - 4 is cut to the same size as the dusting layer and is slot coated with the same suitable adhesive HL 1358 LO and placed on top of the superabsorbent coated dusting layer. This pad forms the absorbent core that is then used in any one of the absorbent articles disclosed above.

Example 7:

A disposable article which is a training pant or a diaper as detailed in any one of the patents listed in Example 6 with the exception that the core has been replaced by the one described below. The core that is included in the article of this example consists of a polypropylene dusting layer (13 gsm) that measures about 140 mm x 390 mm. The dusting layer is slot coated longitudinally down the center 97 mm with a suitable adhesive, e.g., HB Fuller 1358 LO, at an add-on amount of about 5 grams along the length. A layer of superabsorbent material is applied via a print roll to the slot coated dusting layer. A first layer of superabsorbent material, e.g., BASF's ASAP 600Z, is applied such that a profiled superabsorbent material pattern is deposited onto the dusting layer at an add-on amount of about 2.2 grams along the length of the dusting layer in the form of a rectangle that is 90 mm wide in each of the 4 quarters of its length. A sufficient amount, e.g., of a microfiber glue, e.g., Fuller's NW 1151ZP, is intermittently applied via spraying onto the superabsorbent material for immobilization of the material. A second layer of superabsorbent material, e.g., BASF's ASAP 600Z, is applied to a piece of material such as described in any one of Examples 1 - 4 that is the same size as the dusting layer. This material serves as a core cover and is slot coated with a suitable adhesive prior to the deposition of the superabsorbent material. A profiled superabsorbent material pattern is deposited onto the core cover at an add-on amount of about 6.8 grams along the length of the core cover in the form of a rectangle that is 90 mm wide in each of the 4 quarters of its length. A total amount of superabsorbent material is about 9.0 grams. A sufficient amount, e.g., of a microfiber glue, e.g., Fuller's NW 1151ZP, is continuously applied via spraying onto the second layer of superabsorbent material for immobilization of the material. This pad forms the absorbent core that is then used in any one of the absorbent articles disclosed above.

Example 8:

A disposable article which is a training pant or a diaper as detailed in any one of the patents listed in Example 6 with the exception that the core has been replaced by the one described below. The core that is included in the article of this example is 90 mm wide in the 1st and 4th quarters and narrower in the 2nd and 3rd quarters, such as 80 mm wide. The core that is included in the article of this example consists of a polypropylene dusting layer (13 gsm) that measures about 125 mm x 390 mm. The dusting layer is slot coated longitudinally down the center 97 mm with a suitable adhesive, e.g., HB Fuller 1358 LO, at an add-on amount of about 5 grams along the length. A layer of superabsorbent material is applied via a print roll to the slot coated dusting layer. A first layer of superabsorbent material, e.g., BASF's ASAP 600Z, is applied such that a profiled superabsorbent material pattern is deposited onto the dusting layer at an add-on amount of about 3.2 grams along the length of the dusting layer. A sufficient amount, e.g., of a microfiber glue, e.g., Fuller's NW 1151ZP, is intermittently applied via spraying onto the superabsorbent material for immobilization of the material. A second layer of superabsorbent material, e.g., BASF's ASAP 600Z, is applied to a piece of material such as described in any one of Examples 1 - 4 that is the same size as the Dusting Layer. A profiled superabsorbent material pattern is deposited onto the core cover at an add-on amount of about 9.3 grams along the length of the core cover. A total amount of superabsorbent material is about 12.5 grams. A sufficient amount, e.g., of a microfiber glue, e.g., Fuller's NW 1151ZP, is continuously applied via spraying onto the second layer of superabsorbent material for immobilization of the material. This pad forms the absorbent core that is then used in any one of the absorbent articles disclosed above.

Horizontal Full Sheet (HFS) Test Method

The Horizontal Full Sheet (HFS) test method determines the amount of distilled water absorbed and retained by the fibrous structure of the present invention. This method is performed by first weighing a sample of the fibrous structure to be tested (referred to herein as the "dry weight of the sample"), then thoroughly wetting the sample, draining the wetted sample in a horizontal position and then reweighing (referred to herein as "wet weight of the sample"). The absorptive capacity of the sample is then computed as the amount of water retained in units of grams of water absorbed by the sample. When evaluating different fibrous structure samples, the same size of fibrous structure is used for all samples tested.

The apparatus for determining the HFS capacity of fibrous structures comprises the following:

1) An electronic balance with a sensitivity of at least $\pm 0.01$ grams and a minimum capacity of 1200 grams. The balance should be positioned on a balance table and slab to minimize the vibration effects of floor/benchtop weighing. The balance should also have a special balance pan to be able to handle the size of the sample tested (i.e.; a fibrous structure sample of about 11 in. (27.9 cm) by 11 in. (27.9 cm)). The balance pan can be made out of a variety of materials. Plexiglass is a common material used.

2) A sample support rack and sample support cover is also required. Both the rack and cover are comprised of a lightweight metal frame, strung with 0.012 in. (0.305 cm) diameter monofilament so as to form a grid of 0.5 inch squares ($1.27 \text{ cm}^2$). The size of the support rack and cover is such that the sample size can be conveniently placed between the two.

The HFS test is performed in an environment maintained at $23 \pm 1$ °C and $50 \pm 2\%$ relative humidity. A water reservoir or tub is filled with distilled water at $23 \pm 1$ ° C to a depth of 3 inches (7.6 cm).

The fibrous structure sample to be tested is carefully weighed on the balance to the nearest 0.01 grams. The dry weight of the sample is reported to the nearest 0.01 grams. The empty sample support rack is placed on the balance with the special balance pan described above. The balance is then zeroed (tared). The sample is carefully placed on the sample support rack. The support rack cover is placed on top of the support rack. The sample (now sandwiched between the rack and cover) is submerged in the water reservoir. After the sample is submerged for 60 seconds, the sample support rack and cover are gently raised out of the reservoir.

The sample, support rack and cover are allowed to drain horizontally for $120 \pm 5$ seconds, taking care not to excessively shake or vibrate the sample. While the sample is draining, the rack cover is carefully removed and all excess water is wiped from the support rack. The wet sample and the support rack are weighed on the previously tared balance. The weight is recorded to the nearest 0.01g. This is the wet weight of the sample.

The gram per fibrous structure sample absorptive capacity of the sample is defined as (wet weight of the sample - dry weight of the sample). The horizontal absorbent capacity is defined as: absorbent capacity = (wet weight of the sample - dry weight of the sample) / (dry weight of the sample) and has a unit of gram/gram.

Horizontal Rate Capacity (HRC) Method

Horizontal Rate Capacity (HRC) is an absorbency rate test that measures the quantity of water taken up by a paper sample in a two second time period. The value is reported in grams of water per second. The instrument used to carry out the HRC measurement comprises a pump, pressure gauge, inlet shunt, rotometer, reservoir, sump, outlet shunt, water supply tube, sample holder, sample, balance, and tubing. The instrument is illustrated in U.S. Pat. No. 5,908,707 issued to Cabell et al. the disclosure of which is incorporated herein by reference for the purposes of showing the instrument used to carry out the HRC measurement.

In this method, the sample (cut using a 3 in. (7.6 cm) diameter cutting die) is placed horizontally in a holder suspended from an electronic balance. The holder is made up of a lightweight frame measuring approximately 7 in. by 7 in. (17 cm by 17 cm), with lightweight nylon monofilament strung through the frame to form a grid of 0.5 in. (1.27 cm) squares. The nylon monofilament for stringing the support rack should be $0.069. \pm 0.005$ in. ($0.175$ cm. $\pm 0.0127$ cm) in diameter (e.g., Berkley Trilene Line 2 1b test clear). The electronic balance used should be capable of measuring to the nearest 0.001 g. (e.g., Sartorious L420P+).

The sample in the holder is centered above a water supply tube. The water supply is a plastic tube having a 0.312 inch (0.79 cm) inside diameter containing distilled water at 23° $\pm$1° C. The supply tube is connected to a fluid reservoir at zero hydrostatic head relative to the test sample. The water supply tube is connected to the reservoir using plastic (e.g. Tygon.RTM.) tubing. The height of the nylon monofilament of the sample holder is located 0.125 in $\pm$1/64 in. (0.32 cm $\pm$0.04 cm) above the top of the water supply tube.

The water height in the reservoir should be level with the top of the water supply tube. The water in the reservoir is continuously circulated using a water pump circulation rate of 85-93 ml/second using a water pump (e.g., Cole-Palmer Masterflex 7518-02) with #6409-15 plastic tubing. The circulation rate is measured by a rotometer tube (e.g., Cole-Palmer N092-04 having stainless steel valves and float). This circulation rate through the rotometer creates a head pressure of 2.5 $\pm$0.5 psi as measured by an Ashcroft glycerine filled gauge.

Before conducting this measurement, the samples should be conditioned to 23°$\pm$1° C and 50 $\pm$2% Relative Humidity for 2 hours. The HRC test is also performed in these controlled environmental conditions.

To start the absorbent rate measurement, the 3 in. (7.62 cm) sample is placed on the sample holder. Its weight is recorded in 1 second intervals for a total of 5 seconds. The weight is averaged (herein referred to as "Average Sample Dry Weight"). Next, the circulating water is shunted to the sample water supply for 0.5 seconds by shunting through the valve. The weight reading on the electronic balance is monitored. When the weight begins to increase from zero a stop watch is started. At 2.0 seconds the sample water supply is shunted to the inlet of the circulating pump to break contact between the sample and the water in the supply tube.

The shunt is performed by diverting through the valve. The minimum shunt time is at least 5 seconds. The weight of the sample and absorbed water is recorded to the nearest 0.001 g. at time equals 11.0, 12.0, 13.0, 14.0 and 15.0 seconds. The five measurements are averaged and recorded as "Average Sample Wet Weight".

The increase in weight of the sample as a result of water being absorbed from the tube to the sample is used to determine the absorbency rate. In this case, the rate (grams of water per second) is calculated as:

$$\text{(Average Sample Wet Weight-Average Sample Dry Weight)/2 seconds}$$

It is understood by one skilled in the art that the timing, pulsing sequences, and electronic weight measurement can be computer automated.

Liquid Strike-Through Test

The liquid strike- through time is measured using Lister-type strike-through equipment, manufactured by Lenzing AG, Austria. Test procedure is based on standardized WSP (World Strategic Partners) 70.7(05), with the test sample placed on an absorbent pad comprised of ten plies of filter paper (Ahlstrom Grade 989 obtained from Empirical Manufacturing Co., Inc., or equivalent). In a typical experiment, three consecutive 5 ml gushes of test liquid (0.9% saline solution) are applied to a non- woven sample at one minute intervals and the respective strike-through times are recorded without changing the absorbent pad.

In addition to measuring the strike-through time for the first gush, as described in the WSP Method, the test described below does not only measure the first gush hut several subsequent gushes, especially the fifth gush.

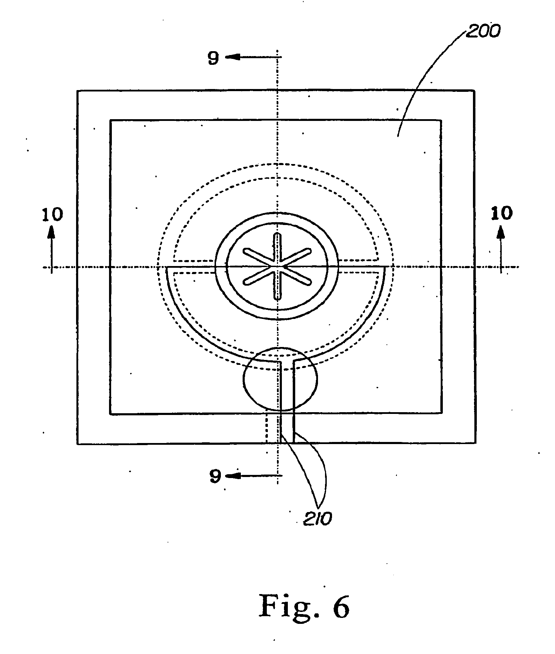

Apparatus-Lister Strike-through Equipment-(i) A Funnel fitted with magnetic valve: Rate of discharge of 25 ml in 3,5 ($\pm$0.25) seconds; (ii) A Strike-through plate: Constructed of 25 mm thick acrylic glass. The total weight of the plate must be 500 g. The electrodes should be of non-corrosive material. The electrodes are set in (4.0 mmx7.0 mm) cross section grooves, cut in the base of the plate and fixed with quick setting epoxy resin. Figures 6, 7, and 8 illustrate a Strike-through plate 200 containing electrodes 210. Figure 6 is a top view of a Strike- through plate 200, whereas Figure 7 is a sectional view along 9-9 of the Strike-through plate 200 of Figure 6. Figure 8 is a sectional perspective view along 10-10 of the Strike-through plate 200 of Figure 6; (iii) Base plate: A square of acrylic glass 125 mm x 125 mm approximately; (iv) Ring stand to support the funnel; (v) Electronic Timer measuring to 0.01 seconds; (vi) Burette with SO ml capacity; and (vii) Core filter paper Ahlstrom Grade 989 or equivalent (average Strike-through time 1.7 s+-0.3 s, dimensions: 10x10 cm).

Procedure: (1) Carefully cut the required number of samples, 12.5 cm x 12.5 cm with touching the sample only at the edge of the sample. (2) Taking 10 plies of Core filter paper place one sample on the set of 10 plies of filter paper on the base plate. The sample should be positioned on the filter paper in such a way that the side of the nonwoven, which is intended to face the user's skin, (when applied in an absorbent article) is uppermost. (3) Place the strike through plate on top with the center of the plate over the center of the test piece. Center the burette and the funnel over the plate. (4) Ensuring that the electrodes are connected to the timer, switch on the timer and set the clock to zero. (5) Fill the burette with saline solution (0.9 wt% NaCl in deionized water). (6) Keep the discharge valve of the funnel closed and run 5.0 ml of liquid (= one gush) from the burette into the funnel. (7) Open the magnetic valve of the funnel to discharge 5.0 ml of liquid. The initial flow of liquid will complete the electrical circuit and start the timer. It will stop when the liquid has penetrated into the pad and fallen below the level of the electrodes in the strike through plate. (8) Record the time indicated on the electronic timer. (9) Wait 60 seconds and repeat steps (4) and (6) to (9) for the second, third and any subsequent gush, with each gush comprising 5 ml of liquid. (e.g., 5ml into funnel, open magnetic valve, etc.) Record the time for the 1st, 2nd, and any subsequent gush in seconds.

Method of Detection of Association of Hydrophilizing Agent and Synthetic Fibers

A nonwoven fibrous structure may be analyzed for the association of synthetic fibers and a hydrophilizing agent in a variety of ways. The fibrous structure may be separated into its component parts which may include synthetic fibers and natural fibers. The synthetic fibers and natural fibers may be separated from each other by any suitable method known to one of ordinary skill in the art.

A method of analyzing the association of synthetic fibers and hydrophilizing agent may include the utilization of the Wilhelmy balance technique. In such a method, the analysis is performed by mounting an individual fiber, such as a synthetic fiber separated from the fibrous structure as discussed above, vertically and measuring the force of water as a function of position as the fiber dips into the water. The contact angle is calculated from the regressed force data and fiber diameter. As an example of such a method, the following table may illustrate the mean contact angle for fibers taken from two handsheets. The numbers presented are an average of three fibers of each sample type in triplicate.. The mean contact angle for the two fibers types is statistically different and may indicate that a hydrophilizing agent has associated with the synthetic fibers of Sample B and has therefore made the fibers more hydrophilic than those of Sample A.

|  | Fiber Diameter ($\mu$m) | Mean Contact Angle | Standard Deviation |
|---|---|---|---|
| Sample A | 14.36 | 73.4° | 2.4° |
| Sample B | 15.04 | 56.4 | 2.8 |

Sample A: About 70% Northern Softwood Kraft cellulose fibers and about 30% CoPET/PET fibers.

Sample B: About 70% Northern Softwood Kraft cellulose fibers and about 30% CoPET/PET fibers and about 40ppm TexCare™ SRN-240.

Another method for analyzing the association of synthetic fibers and hydrophilizing agent may include the separation of the fibers as described. The synthetic fibers may then undergo an extraction process, such as a solvent extraction, to remove any surface coatings, elements, contaminants, etc from the synthetic fibers to result in "clean" synthetic fibers. The solvent extract may be analyzed by any suitable method known to one of ordinary skill, including, but not limited to, liquid chromatography, mass spectrometry, static time-of-flight secondary ion mass spectrometry, etc. to determine the presence of a hydrophilizing agent, such as a hydrophilizing agent comprising a polyester segment. The synthetic fibers and the hydrophilzing agent may be analyzed to determine the actual synthetic fiber and the actual hydrophilizing agent present in the fibrous structure. The presence of both synthetic fibers and hydrophilizing agent characterizes the association of the synthetic fibers with the hydrophilizing agent.

Method of Determining Durability of Association of Hydrophilizing Agent and Synthetic Fibers

Synthetic fibers may be analyzed for the durability of the association of the synthetic fibers and a hydrophilizing agent. A method for determining the durability of the association may relate to the wettability of the synthetic fibers. Contact angle measurement of a liquid, such as water, in contact with the synthetic fibers may provide for a determination of the durability of the association between a synthetic fiber and a hydrophilizing agent. A wettable synthetic fiber may demonstrate the association of the synthetic fiber and a hydrophilizing agent. A demonstration of the wettability of the synthetic fiber following multiple washings may demonstrate the durability of the association of the synthetic fiber and a hydrophilizing agent.

The synthetic fibers may be dried at about 80°C in an air flow oven for about 24 hours. The synthetic fibers may be placed in a beaker and washed in warm water (about 60°C) for two hours with gentle stirring to remove any residual process aids. The ratio of fibers to water volume may be about 1:200. After washing, the fibers may be collected and dried overnight at room temperature. The synthetic fibers may be separated into four groups, with each group weighing about 36 grams, and placed in an air flow over for about 10 hour. Four aliquots, each about 5 grams, may be separated out and may be treated with a hydrophilizing agent and a surfactant at two varying levels, such as 40 ppm and 400 ppm. Therefore, one aliquot of 5 grams of synthetic fibers may be soaked in about 40 ppm of a hydrophilizing agent for about 10 minutes. A second aliquot of 5 grams of synthetic fibers may be soaked in about 400 ppm of a hydrophilizing agent for about 10 minutes. A third aliquot of about 5 grams of synthetic fibers may be soaked in about 40 ppm of a surfactant for about 10 minutes. A fourth aliquot of 5 grams of synthetic fibers may be soaked in about 400 ppm of a surfactant for about 10 minutes. The ratio of each treatment group of synthetic fibers to treatment is 5g : 100 ml of solution. The four groups of synthetic fibers may be dried post-treatment at room temperature. After drying, the four groups of synthetic fibers may be subjected to about 10 minutes of water washing using double distilled water at about 45°C.

A method of analyzing the association of synthetic fibers and hydrophilizing agent or surfactant may include the utilization of the Wilhelmy balance technique. In such a method, the analysis is performed by mounting an individual fiber vertically and measuring the force of water as a function of position as the fiber dips into the water. The contact angle is calculated from the regressed force data and fiber diameter. As an example of such a method, the following table may illustrate the mean contact angle for synthetic fibers that have been subjected to the above various treatments and wash steps. The numbers presented are an average of two fibers of each sample type in triplicate.

| Sample | Treatment | Water Wash | Mean Contact Angle (°) | Standard Deviation (°) | Fiber Diameter (μm) |
|---|---|---|---|---|---|
| 1 | 40 ppm Hydrophilizing Agent | None | 54.9 | 1.8 | 13.40 |
| 2 | 400 ppm Hydrophilizing Agent | None | 52.2 | 1.7 | 15.14 |
| 3 | 40 ppm Surfactant | None | 71.3 | 1.9 | 14.06 |
| 4 | 400 ppm Surfactant | None | 60.9 | 2.3 | 14.78 |
| 5 | 40 ppm Hydrophilizing Agent | 10 Minutes | 62.2 | 1.9 | 13.45 |
| 6 | 400 ppm Hydrophilizing Agent | 10 Minutes | 66.1 | 1.8 | 15.13 |
| 7 | 40 ppm Surfactant | 10 Minutes | 80.0 | 2.1 | 14.78 |
| 8 | 400 ppm Surfactant | 10 Minutes | 82.4 | 1.8 | 13.73 |
| 9 | No treatment | 10 Minutes | 72.2 | 2.2 | 14.88 |

The synthetic fibers used for each sample are bicomponent fibers of CoPET/PET. The hydrophilizing agent utilized is TexCare™ SRN-240 and the surfactant utilized is Triton-X 100 as available from The Dow Chemical Company.

As demonstrated by the above table, the synthetic fibers treated with a hydrophilizing agent may demonstrate lower contact angles and, therefore, durable wettability post washing when compared to the post washing synthetic fibers treated with a surfactant.

Sustainable Wettability

A nonwoven fibrous structure may be analyzed for sustainable wettability in the following manner. The sample fibrous structure may be placed on an absorbent pad. Multiple insults of test liquid may be applied to the fibrous structure at timed intervals. Each insult of liquid may be considered as a strike-through. The strike-through times may then be recorded without changing the absorbent pad.

In one example, a nonwoven fibrous structure exhibits sustainable wettability if after saturating the fibrous structure with water (test liquid) multiple times (at least ten (10) times or more), the fibrous structure still exhibits an HRC value of at least about 0.1 g/sec and/or at least about 0.2 g/sec and/or at least about 0.3 g/sec and/or at least about 0.4 g/sec and/or at least about 0.5 g/sec.

**Claims**

1. An absorbent article **characterized in that** said absorbent article comprises a nonwoven fibrous structure comprising a plurality of synthetic fibers, wherein one or more of said synthetic fibers comprises a polymer, and a hydrophilizing agent wherein said polymer and said hydrophilizing agent comprise a durable association; and wherein said polymer and said hydrophilizing agent comprise complementary segments that are associated with one another; and wherein said complementary segment of said polymer comprises a polyester segment and said complementary segment of said hydrophilizing agent comprises a polyester segment; and wherein said fibrous structure is a component of said article selected from the group consisting of core cover, acquisition layer, dusting layer and combinations thereof.

2. The absorbent article according to Claim 1 wherein said polyester segment comprises a polyethylene terephthalate.

3. The absorbent article according to any of the preceding claims wherein said polymer comprises a material selected from the group consisting of polyesters, polyamides, polyhydroxyalkanoates, polysaccharides, and combinations thereof.

4. The absorbent article according to any of the preceding claims wherein said hydrophilizing agent comprises a material selected from the group consisting of polyester, poly(ethoxylate), polyethylene oxide, polyoxyethylene, polyethylene glycol, polypropylene glycol, terephthalate, polypropylene oxide, polyethylene terephthalate, polyoxyethylene terephthalate, ethoxylate siloxane and combinations thereof.

5. The absorbent article according to any of the preceding claims wherein said hydrophilizing agent comprises from about 1 to about 15 ethoxylated unites.

6. The absorbent article according to any of the preceding claims wherein said fibrous structure further comprises a plurality of natural fibers.

7. The absorbent article according to any of the preceding claims wherein said fibrous structure further comprises a binder material.

8. The absorbent article according to any of the preceding claims wherein said fibrous structure is a core cover.

9. The absorbent article according to any of the preceding claims wherein said synthetic fibers exhibit a durable wettability.

10. The absorbent article according to any of the preceding claims wherein said synthetic fibers comprise a mean contact angle of less than about 72° and after a 10 minute water wash said mean contact angle remains below about 72°.

**Patentansprüche**

1. Absorptionsartikel, **dadurch gekennzeichnet, dass** der Absorptionsartikel eine Vliesfaserstruktur umfasst, die mehrere synthetische Fasern umfasst, wobei eine oder mehrere der synthetischen Fasern ein Polymer und ein Hydrophilisierungsmittel umfasst, wobei das Polymer und das Hydrophilisierungsmittel eine dauerhafte Assoziierung umfassen; und wobei das Polymer und das Hydrophilisierungsmittel komplementäre Segmente umfassen, die miteinander assoziiert sind; und wobei das komplementäre Segment des Polymers ein Polyestersegment umfasst und das komplementäre Segment des Hydrophilisierungsmittels ein Polyestersegment umfasst; und wobei die Faserstruktur ein Bestandteil des Artikels ist, der ausgewählt ist aus der Gruppe bestehend aus Kernabdeckung, Aufnahmeschicht, Stäubeschicht und Kombinationen davon.

2. Absorptionsartikel nach Anspruch 1, wobei das Polyestersegment ein Polyethylenterephthalat umfasst.

3. Absorptionsartikel nach einem der vorstehenden Ansprüche, wobei das Polymer ein Material umfasst, das ausgewählt ist aus der Gruppe bestehend aus Polyestern, Polyamiden, Polyhydroxyalkanoaten, Polysacchariden und Kombinationen davon.

4. Absorptionsartikel nach einem der vorstehenden Ansprüche, wobei das Hydrophilisierungsmittel ein Material umfasst, das ausgewählt ist aus der Gruppe bestehend aus Polyester, Poly(ethoxylat), Polyethylenoxid, Polyoxyethylen, Polyethylenglycol, Polypropylenglycol, Terephthalat, Polypropylenoxid, Polyethylenterephthalat, Polyoxyethylenterephthalat, Ethoxylatsiloxan und Kombinationen davon.

5.  Absorptionsartikel nach einem der vorstehenden Ansprüche, wobei das Hydrophilisierungsmittel von etwa 1 bis etwa 15 ethoxylierte Einheiten umfasst.

6.  Absorptionsartikel nach einem der vorstehenden Ansprüche, wobei die Faserstruktur ferner mehrere Naturfasern umfasst.

7.  Absorptionsartikel nach einem der vorstehenden Ansprüche, wobei die Faserstruktur ferner ein Bindemittelmaterial umfasst.

8.  Absorptionsartikel nach einem der vorstehenden Ansprüche, wobei die Faserstruktur eine Kernabdeckung ist.

9.  Absorptionsartikel nach einem der vorstehenden Ansprüche, wobei die synthetischen Fasern eine dauerhafte Benetzbarkeit aufweisen.

10. Absorptionsartikel nach einem der vorstehenden Ansprüche, wobei die synthetischen Fasern einen mittleren Kontaktwinkel von weniger als etwa 72° umfassen und der mittlere Kontaktwinkel nach einer 10-minütigen Wasserspülung unter etwa 72° bleibt.


**Revendications**

1.  Article absorbant **caractérisé en ce que** ledit article absorbant comprend une structure fibreuse non tissée comprenant une pluralité de fibres synthétiques, dans lequel une ou plusieurs desdites fibres synthétiques comprennent un polymère, et un agent d'hydrophilisation dans lequel ledit polymère et ledit agent d'hydrophilisation comprennent une association durable ; et dans lequel ledit polymère et ledit agent d'hydrophilisation comprennent des segments complémentaires qui sont associés l'un à l'autre ; et dans lequel ledit segment complémentaire dudit polymère comprend un segment de polyester et ledit segment complémentaire dudit agent d'hydrophilisation comprend un segment de polyester ; et dans lequel ladite structure fibreuse est un composant dudit article choisi dans le groupe constitué d'une protection d'âme, une couche de recueil, une couche de saupoudrage et leurs combinaisons.

2.  Article absorbant selon la revendication 1, dans lequel ledit segment de polyester comprend un polyéthylène téréphtalate.

3.  Article absorbant selon l'une quelconque des revendications précédentes, dans lequel ledit polymère comprend un matériau choisi dans le groupe constitué de polyesters, polyamides, polyhydroxyalcanoates, polysaccharides, et leurs combinaisons.

4.  Article absorbant selon l'une quelconque des revendications précédentes, dans lequel ledit agent d'hydrophilisation comprend un matériau choisi dans le groupe constitué de polyester, poly(éthoxylate), oxyde de polyéthylène, polyoxyéthylène, polyéthylène glycol, polypropylène glycol, téréphtalate, oxyde de polypropylène, polyéthylène téréphtalate, polyoxoéthylène téréphtalate, éthoxylate siloxane et leurs combinaisons.

5.  Article absorbant selon l'une quelconque des revendications précédentes, dans lequel ledit agent d'hydrophilisation comprend d'environ 1 à environ 15 motifs éthoxylés.

6.  Article absorbant selon l'une quelconque des revendications précédentes, dans lequel ladite structure fibreuse comprend en outre une pluralité de fibres naturelles.

7.  Article absorbant selon l'une quelconque des revendications précédentes, dans lequel ladite structure fibreuse comprend en outre un matériau de type liant.

8.  Article absorbant selon l'une quelconque des revendications précédentes, dans lequel ladite structure fibreuse est une protection d'âme.

9. Article absorbant selon l'une quelconque des revendications précédentes, dans lequel lesdites fibres synthétiques présentent une mouillabilité durable.

10. Article absorbant selon l'une quelconque des revendications précédentes, dans lequel lesdites fibres synthétiques comprennent un angle de contact moyen inférieur à environ 72° et après un lavage à l'eau de 10 minutes ledit angle de contact moyen demeure inférieur à environ 72°.

Fig. 1

Fig. 2

Fig. 3

Fig. 4

Fig. 5

Fig. 6

**Fig. 7**

Fig. 8

## REFERENCES CITED IN THE DESCRIPTION

### Patent documents cited in the description

- EP 0438114 A1
- GB 2269603 A
- WO 9405243 A
- US 5356405 A
- US 4981557 A
- US 20040154768 A
- US 20040157524 A
- US 4588457 A
- US 5397435 A
- US 5405501 A
- US 3860003 A
- US 5151029 A
- US 5221274 A
- US 5569234 A
- US 6004306 A
- US 3929135 A
- US 4324246 A
- US 4342314 A
- US 4463045 A
- US 5006394 A
- US 4609518 A
- US 4629643 A
- US 5607760 A
- US 5609587 A
- US 5635191 A
- US 5643588 A
- US 5968025 A
- US 6716441 B
- WO 9524173 A
- US 4892536 A
- US 4990147 A
- US 5037416 A
- US 5269775 A
- US 4515595 A
- US 4695278 A
- US 4909803 A
- US 5151092 A
- US 5167897 A
- US 5143679 A
- US 5156793 A
- US 5705013 A
- US 5683533 A
- US 5580411 A
- US 5137537 A
- US 5599335 A
- US 5562646 A
- US 5669894 A
- US 6790798 B
- US 20040158212 A1
- US 20040097895 A1
- US 758375 A
- US 10758138 B
- US 6627786 B
- WO 9822279 A
- US 4956447 A
- US 3893929 A
- US 3959230 A
- US 3962152 A
- US 4968451 A
- US 4711730 A
- US 4702857 A
- US 4721580 A
- US 4877896 A
- US 5415807 A
- US 4427557 A
- US 4861512 A
- US 5574179 A
- US 5843878 A
- US 4632207 A
- US 4704115 A
- US 4795454 A
- US 4900317 A
- US 5085654 A
- US 5492751 A
- US 6476288 B
- US 6627787 B
- US 5507760 A
- US 6118041 A
- US SIRH1630 A
- US 5908707 A, Cabell